# EUROPEAN PATENT SPECIFICATION

(11) **EP 3 380 453 B1**
(45) Date of publication and mention of the grant of the patent: **30.12.2020**
(21) Application number: 16813183.7
(22) Date of filing: 30.11.2016
(51) Int. Cl.: C07C 319/08, C07C 323/12, B01J 19/24

(54) **BETA-MERCAPTOETHANOL SYNTHESIS**
SYNTHESE VON BETA-MERCAPTOETHANOL
SYNTHÈSE DE BÊTA-MERCAPTOÉTHANOL

(30) Priority: 04.12.2015 US 201514959328
(43) Date of publication of application: 03.10.2018
(60) Divisional application: 19197412.0 / 3 597 635
(73) Proprietor: Chevron Phillips Chemical Company LP, The Woodlands, Texas 77380 (US)
(72) Inventor: HASENBERG, Daniel, Kingwood, Texas 77345 (US); PAUWELS, Alex, 2100 Deurne (BE)
(74) Representative: Privett, Marianne Alice Louise
(86) International application number: PCT/US2016/064133
(87) International publication number: WO 2017/095873

(56) References cited:
- DE-A1- 2 129 162
- DATABASE WPI Week 201552 Thomson Scientific, London, GB; AN 2015-408130 XP002766859, & RU 2 556 859 C1 (NIZHNEKAMSKNEFTEKHIM PUBLIC STOCK CO) 20 July 2015 (2015-07-20)
- "Methyl t-butyl ether prepn. - by passing mixt. of isobutylene and methanol through adiabatic reactor and then to reacting distn. tower", DERWENT, 5 April 1985 (1985-04-05), XP002278112,
- ANDRUSKI R ET AL: "Studies on the synthesis of 2-mercaptoethanol", ORGANIKA, INSTYTUT PRZEMYSLU ORGANICZNEGO, POLAND, 1 January 1979 (1979-01-01), pages 15-23, XP009182190, ISSN: 0137-9933

## Description

### CROSS-REFERENCE TO RELATED APPLICATIONS

Not applicable.

### STATEMENT REGARDING FEDERALLY SPONSORED RESEARCH OR DEVELOPMENT

Not applicable.

### REFERENCE TO A MICROFICHE APPENDIX

Not applicable.

### FIELD

This disclosure relates to the synthesis of beta-mercaptoethanol via the conversion of ethylene oxide.

### BACKGROUND

Beta-mercaptoethanol (BME) is mainly used in poly(vinyl chloride) (PVC) production as an intermediate for the synthesis of PVC heat stabilizers and as a process regulator in PVC manufacturing. BME is a common reducing agent and is also used as a component in corrosion inhibitors, as a processing aid for the leather industry, and as a laboratory chemical in biochemical applications. BME is generally produced via reaction of ethylene oxide (EO) with hydrogen sulfide (H₂S) in a continuous stirred-tank reactor (CSTR) in the presence of excess hydrogen sulfide (H₂S). However, in a CSTR, conversion of ethylene oxide to BME is limited, and undesirable byproducts such as thiodiglycol and other heavy products are also produced. Thus, there is an ongoing need for developing methods for producing BME

DATAPASE WPI, Week 201552 discloses an adiabatic reactor whereby isoamylene is diluted before feeding to a reactor. RU2556859 discloses a method for increasing the yield of isoprene by increasing the conversion of isoamylenes, as well as reducing the specific consumption of water vapor. DERWENT "Methyl t-butyl ether prepn. - by passing mixt. of iosbutylene and methanol through adiabatic reactor and then to reacting distn. tower" (1985-04-05) discloses an adiabatic reactor whereby a mixture of isobutylene and methanol is fed to a reactor. DE2129162 discloses a reactor whereby hydrogen sulfide and ethylene oxide are mixed and fed to a reactor which is filled with an ion exchange resin. Andruski R et al. "Studies on the synthesis of 2-mercaptoethanol" (1979) discloses one possible reaction of ethylene oxide and hydrogen sulfide to yield beta mercaptoethanol.

### SUMMARY

Disclosed is a process comprising reacting, in a reactor having a fixed bed containing a solid catalyst comprising a heterogeneous ion exchange resin, hydrogen sulfide and ethylene oxide in the presence of the solid catalyst to yield a reaction product comprising beta-mercaptoethanol, wherein during steady state operation of the reactor, the hydrogen sulfide and the ethylene oxide are present in a mole ratio in a range of 9:1 to 20:1.

Also disclosed, but not part of the presently claimed invention, is a reactor system which includes a reactor; an ethylene oxide stream feeding ethylene oxide to the reactor; a hydrogen sulfide stream feeding hydrogen sulfide to the reactor; a fixed bed containing a solid catalyst placed in the reactor; and an effluent stream flowing from the reactor, wherein the effluent stream comprises a reaction product comprising beta-mercaptoethanol yielded in the reactor by a reaction of hydrogen sulfide and ethylene oxide in presence of the solid catalyst; wherein the solid catalyst comprises a heterogeneous ion exchange resin, and wherein the hydrogen sulfide and the ethylene oxide are present in the reactor during steady state operation in a mole ratio ranging from 9:1 to 20:1.

### BRIEF DESCRIPTION OF THE DRAWINGS

For a detailed description of the preferred embodiments of the disclosed processes, reference will now be made to the accompanying drawing in which:

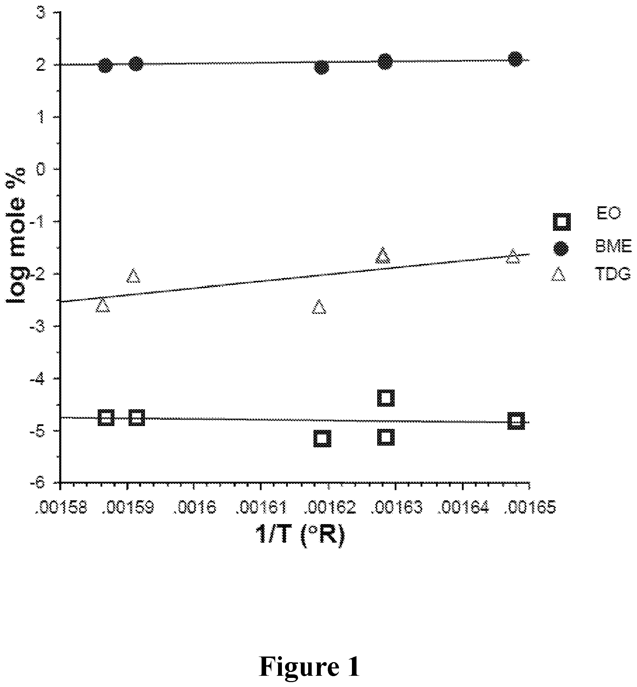

Figure 1 displays an Arrhenius plot of activation energies for a LEWATIT® MP 62 resin-catalyzed beta-mercaptoethanol synthesis from ethylene oxide and hydrogen sulfide.

### DETAILED DESCRIPTION OF THE EMBODIMENTS

Disclosed herein are embodiments of processes to produce beta-mercaptoethanol (BME) via conversion of ethylene oxide (EO) to beta-mercaptoethanol in an excess of hydrogen sulfide (H₂S) and in the presence of a solid catalyst. The ethylene oxide reacts with hydrogen sulfide in a reactor comprising a fixed bed containing a solid catalyst comprising a heterogeneous ion exchange resin. In some embodiments, the solid catalyst can have weakly basic active groups to allow for conversion of ethylene oxide by hydrogen sulfide.

The following definitions are provided in order to aid those skilled in the art in understanding the detailed description of the present invention. Unless otherwise defined herein, scientific and technical terms used in connection with the present invention shall have the meanings that are commonly understood by those of ordinary skill in the art to which this invention belongs. Further, unless otherwise required by context, singular terms shall include pluralities and plural terms shall include the singular.

Unless explicitly stated otherwise in defined circumstances, all percentages, parts, ratios, and like amounts used herein are defined by weight.

Further, certain features of the present invention which are, for clarity, described herein in the context of separate embodiments, may also be provided in combination in a single embodiment. Conversely, various features of the invention that are, for brevity, described in the context of a single embodiment, may also be provided separately or in any sub-combination.

If a term is used in this disclosure but is not specifically defined herein, the definition from the IUPAC Compendium of Chemical Terminology, 2nd Ed (1997), can be applied, as long as that definition does not conflict with any other disclosure or definition applied herein, or render indefinite or non-enabled any claim to which that definition is applied.

As used herein, beta-mercaptoethanol (BME) can also be referred to by various synonyms including, 2-sulfanylethanol (IUPAC name), 2-hydroxy-1-ethanethiol, thioglycol, 2-mercaptoethanol, 2-thioethanol, 2-hydroxyethyl mercaptan, and others. As used herein, ethanedithiol can also be referred to by various synonyms, including, ethane-1,2-dithiol (IUPAC name), 1,2-ethanedithiol, 1,2-dimercaptoethane, dithioglycol, ethylene mercaptan, and others. As used herein, thiodiglycol can also be referred to by various synonyms including, 2-(2-hydroxyethylsulfanyl) ethanol (IUPAC name), 2,2'-thiodiethanol, thiodiethanol, thiodiethylene glycol, and others.

Regarding claim transitional terms or phrases, the transitional term "comprising," which is synonymous with "including," "containing," "having," or "characterized by," is inclusive or open-ended and does not exclude additional, unrecited elements or method steps. The transitional phrase "consisting of' excludes any element, step, or ingredient not specified in the claim. The transitional phrase "consisting essentially of' limits the scope of a claim to the specified materials or steps and those that do not materially affect the basic and novel characteristic(s) of the claim. A "consisting essentially of' claim occupies a middle ground between closed claims that are written in a "consisting of' format and fully open claims that are drafted in a "comprising" format. Absent an indication to the contrary, describing a compound or composition as "consisting essentially of' is not to be construed as "comprising," but is intended to describe the recited component that includes materials which do not significantly alter the composition or method to which the term is applied. For example, a feedstock consisting essentially of a material A can include impurities typically present in a commercially produced or commercially available sample of the recited compound or composition. When a claim includes different features and/or feature classes (for example, a method step, feedstock features, and/or product features, among other possibilities), the transitional terms comprising, consisting essentially of, and consisting of apply only to the feature class to which it is utilized, and it is possible to have different transitional terms or phrases utilized with different features within a claim. For example, a method can comprise several recited steps (and other non-recited steps), but utilize a catalyst system consisting of specific components; alternatively, consisting essentially of specific components; or alternatively, comprising the specific components and other non-recited components.

In this disclosure, while systems, processes, and methods are often described in terms of "comprising" various components, devices, or steps, the systems, processes, and methods can also "consist essentially of' or "consist of' the various components, devices, or steps, unless stated otherwise.

The term "about" means that amounts, sizes, formulations, parameters, and other quantities and characteristics are not and need not be exact, but may be approximate and/or larger or smaller, as desired, reflecting tolerances, conversion factors, rounding off, measurement error, and other factors known to those of skill in the art. In general, an amount, size, formulation, parameter or other quantity or characteristic is "about" or "approximate" whether or not expressly stated to be such. The term "about" also encompasses amounts that differ due to different equilibrium conditions for a composition resulting from a particular initial mixture. Whether or not modified by the term "about," the claims include equivalents to the quantities. The term "about" may mean within 10% of the reported numerical value, preferably within 5% of the reported numerical value.

Although any methods, devices, and materials similar or equivalent to those described herein can be used in the practice or testing of the invention, the typical methods, devices, and materials are herein described.

The present invention, provides a process for producing beta-mercaptoethanol (BME) comprising reacting, in a reactor having a fixed bed containing a solid catalyst comprising a heterogeneous ion exchange resin, hydrogen sulfide and ethylene oxide in the presence of the solid catalyst to yield a reaction product comprising BME, wherein during steady state operation of the reactor, the hydrogen sulfide and the ethylene oxide are present in a mole ratio in a range of 9:1 to 20:1.

The reactor comprises any suitable fixed bed catalytic reactor. Generally, a fixed bed catalytic reactor contains a fixed bed of solid catalyst, wherein the solid catalyst does not move with respect to a fixed or immobile reference point on a reactor body. The solid catalyst can be retained in the fixed bed by any suitable methodology, such as for example by employing retaining screens.

In an embodiment, the fixed bed catalytic reactor can be a continuous flow reactor, such as a plug flow reactor (PFR). Generally, a PFR, also known as a flow tube reactor, comprises a fluid flowing through the reactor as a series of infinitely thin coherent "plugs," each plug having a uniform composition, traveling in the axial direction of the flow tube reactor. In PFRs, it is assumed that as a plug flows through the reactor, the fluid is perfectly mixed in the radial direction, but not in the axial direction (forwards or backwards).

In an embodiment, the reactor can be an adiabatic reactor. Generally, adiabatic reactors do not provide for heat exchange between the interior of the reactor (e.g., catalyst, catalyst bed, reactants, etc.) and the surroundings of the reactor. In an embodiment, no internal and/or external cooling source is used to cool the reactor. For purposes of the disclosure herein, the term "internal cooling" excludes evaporative cooling due to (e.g., owing to) hydrogen sulfide converting from a liquid phase to a vapor phase, thereby absorbing a heat of reaction. In embodiments, cooling equipment which are not used for external cooling include heat exchangers and reactor jackets. In embodiments, cooling equipment which are not used for internal cooling include internal heat exchange elements (e.g., heat exchange elements that would be placed inside the reactor) that can contain a heat transfer medium (i.e., a heat transfer fluid such as water, mineral oil(s), synthetic or organic based fluid(s), or combinations thereof). Further, for purposes of the disclosure herein, the terms "internal cooling" and "external cooling" refer to cooling by heat exchange based on energy input outside of the reactor. The adiabatic reactor as used herein can be a "self-cooling" reactor via converting (e.g., evaporating) hydrogen sulfide from a liquid phase to a vapor phase. As will be appreciated by one of skill in the art, and with the help of this disclosure, the reaction of ethylene oxide with hydrogen sulfide to produce BME is exothermic, as will be discussed in more detail later herein.

In an embodiment, the solid catalyst can comprise any suitable basic solid catalyst. The solid catalyst comprises a heterogeneous ion exchange resin. Generally, heterogeneous catalysts refer to catalysts that are in a different phase than the reactants for which they catalyze a reaction. For example, the heterogeneous ion exchange resin can be a solid, while ethylene oxide and hydrogen sulfide can be in a liquid (or gas) phase contacting the solid resin catalyst. Generally, ion exchange resins are polymers containing loosely held ions, and the polymers are capable of exchanging such ions with other ions in solutions that come in contact with the polymer. These ion exchanges can take place without any physical alteration to the ion exchange material. Ion exchange resins are insoluble acids or bases that have salts which are also insoluble, and this enables exchange of either positively charged ions (e.g., basic ion exchange resins or cation exchangers) or negatively charged ions (e.g., acidic ion exchange resins or anion exchangers).

Ion exchange resins generally consist of a crosslinked polymer matrix with a relatively uniform distribution of active ion exchange sites throughout the structure. In an embodiment, the polymer matrix suitable for use in the present disclosure as part of the ion exchange resin comprises polystyrene, copolymers of styrene and divinylbenzene (DVB), polyacrylic esters, polyethyleneimine, polyethylene, polypropylene, copolymers thereof, or combinations thereof.

In some embodiments, the solid catalyst can have basic active groups. In other embodiments, the solid catalyst can have weakly basic active groups. For purposes of the disclosure herein, the term "active group" or "active groups" when used to describe the catalyst refers to groups active for ion exchange. In an embodiment, the basic active groups can comprise quaternary ammonium groups. In an embodiment, the weakly basic active groups can comprise primary amine groups, secondary amine groups, tertiary amine groups, or combinations thereof. As will be appreciated by one of skill in the art, and with the help of this disclosure, primary, secondary and tertiary amine groups show weak basicity, and as such can be referred to as weakly basic active groups.

Nonlimiting examples of weakly basic active groups suitable for use in the current disclosure as part of the solid catalyst include alkyl amines, monoalkyl amine, dialkyl amine, dimethyl amine, trialkyl amines, or combinations thereof.

In an embodiment, the solid catalyst can comprise a styrene-DVB copolymer with weakly basic active groups comprising alkyl amines.

Commercial examples of solid catalysts suitable for use in the current disclosure include AMBERLYST® A21 resin which is an industrial grade weakly basic polymeric resin in bead form commercially available; and LEWATIT® MP 62 resin which is weakly basic, and macroporous anion exchange resin with tertiary amine groups (monofunctional) commercially available.

In an embodiment, the fixed bed of the solid catalyst can further comprise a chemically inert solid diluent or bulking material. Nonlimiting examples of chemically inert solid diluents suitable for use in the current disclosure as part of the fixed bed include fused alumina, polystyrene, copolymers of styrene and divinylbenzene (DVB), polyacrylic esters, polyethylene, polypropylene, copolymers thereof, or combinations thereof.

In some embodiments, the fixed bed of the solid catalyst can comprise a single zone (e.g., single catalytic zone) comprising the solid catalyst and optionally the chemically inert solid diluent, for example the bed materials being uniformly mixed.

In other embodiments, the fixed bed of the solid catalyst can comprise two or more zones (e.g., catalytic zones), wherein each zone comprises the solid catalyst and optionally the chemically inert solid diluent. When the fixed bed of the solid catalyst comprises two or more zones (e.g., catalytic zones), the solid catalyst can be distributed along the fixed bed according to a volumetric concentration gradient. For example, a volumetric concentration of the solid catalyst can increase from an entry point into a catalyst bed (e.g., reagents entry point(s)) to an exit point from the catalyst bed (e.g., reaction product exit point), e.g., a volumetric concentration of the solid catalyst can increase along a catalyst bed in the direction of the flow through a flow reactor. In some embodiments, an increase in the volumetric concentration of the solid catalyst can be continuous along a length of the fixed bed or zone thereof. In other embodiments, the increase in the volumetric concentration of the solid catalyst can be step-wise across a length of the fixed bed or zone thereof. As another example, a volumetric concentration of the solid catalyst can decrease from an entry point into a catalyst bed (e.g., reagents entry point(s)) to an exit point from the catalyst bed (e.g., reaction product exit point), e.g., a volumetric concentration of the solid catalyst can decrease along a catalyst bed in the direction of the flow through a flow reactor. As yet another example, a volumetric concentration of the solid catalyst can decrease along some regions or zones of the catalyst bed, increase along other regions of the catalyst bed, and stay the same along yet other regions of the catalyst bed. The amount of chemically inert solid diluent can likewise be adjusted or varied across the reactor bed geometry (e.g., along a length of the fixed bed or zone thereof) to provide a desired solid catalyst volumetric concentration profile.

In some embodiments, the solid catalyst can be distributed evenly along the fixed bed, e.g., the volumetric concentration of the catalyst can stay the same along a length of the fixed bed or zone thereof, for example uniformly mixed with chemically inert solid diluent, if present.

In an embodiment, the fixed bed of the solid catalyst can comprise a top zone, a middle zone, and a bottom zone, wherein the flow in the reactor can be from a top zone towards a bottom zone, and wherein the middle zone can be located between the top zone and the bottom zone. In such embodiment, the top zone comprises both a solid catalyst and a chemically inert solid diluent, wherein an amount of the chemically inert solid diluent by volume is greater (e.g., greater than 50%, 55%, 60%, 65%, 70%, 75%, 80%, 85%, 90%, 95%, or 99%, based on a total volume of the top zone) than an amount of the solid catalyst by volume in the top zone; the middle zone comprises both a solid catalyst and a chemically inert solid diluent, wherein an amount of the chemically inert solid diluent by volume is about the same (e.g., about 45/55, about 50/50, about 55/45, based on volumetric ratios of chemically inert solid diluent to solid catalyst in the middle zone) as an amount of the solid catalyst by volume in the middle zone; and wherein the bottom zone comprises a solid catalyst and a lesser amount by volume (e.g., less than 50%, 45%, 40%, 35%, 30%, 25%, 20%, 15%, 10%, 5%, 1%, or 0%, based on a total volume of the bottom zone) of the chemically inert solid diluent.

In an embodiment, the hydrogen sulfide can be fed to the reactor in a downflow orientation. As the hydrogen sulfide travels along the length of the fixed bed of the reactor, a temperature of the fixed bed can increase along the length of the reactor in the direction of the flow, and some of the hydrogen sulfide will convert from a liquid phase to a gas phase (e.g., a portion of the liquid hydrogen sulfide will vaporize), thereby absorbing a portion of the reaction heat and controlling the temperature inside the reactor. The reactor is designed as an adiabatic packed bed reactor with no internals for transferring the heat of reaction. The diameter is sufficiently large so that the heat transferred through the outside walls is small relative to the heat released by the reaction. The EO and H₂S feeds enter the top of the reactor as a liquid. Once entering the catalyst bed the heat of reaction converts at least a portion of the liquid H₂S into a gas. Then the reactor is operated as a typical trickle-bed reactor with liquid and gas flow concurrently flowing downward through the packed bed while reaction takes place. Co-current downward flow provides the best hydrodynamic flow patterns with the catalyst particle sizes of interest and avoids the need of internals to keep the catalyst bed intact inside of the reactor.

The disclosed embodiments include a relatively low temperature and low pressure for the conversion of ethylene oxide to BME by reacting the ethylene oxide with hydrogen sulfide in the presence of a solid catalyst under conditions as described herein. In an embodiment, hydrogen sulfide and ethylene oxide can react in a liquid phase, wherein the liquid phase can contact the solid catalyst. As will be appreciated by one of skill in the art, and with the help of this disclosure, the relatively low temperature and low pressure employed in the disclosed process are adequate to allow for the reactants to be in a liquid phase.

According to the disclosed embodiments, ethylene oxide and hydrogen sulfide can be reacted in liquid phase at a temperature in the approximate range of from 30 °C to 80 °C in the presence of a heterogeneous ion exchange catalyst and at a molar ratio of hydrogen sulfide to ethylene oxide in the range of from 9:1 to 20:1, as previously described herein.

In an embodiment, a process for producing BME can be performed at a temperature (e.g., the reactor can be characterized by a temperature) in a range of from 30 °C to 80 °C, alternatively from 30 °C to 51 °C, alternatively from 35 °C to 75 °C, or alternatively from 40 °C to 70 °C. Generally, ion exchange resins can deactivate upon exposure to high temperatures, and as such a catalyst longevity (e.g., length of time that a catalysts does not significantly lose catalytic activity) of the heterogeneous ion exchange resin catalyst under reactor operating conditions can be extended by maintaining the temperature in the reactor under 80 °C.

In an embodiment, the fixed bed of the reactor can have a weight average bed temperature (WABT) of from 65 °C to 70 °C, alternatively from 65.5 °C to 69.5 °C, or alternatively from 66 °C to 69 °C. Generally, the WABT is calculated for catalyst beds that do not have a uniform catalyst distribution across the reactor (e.g., multiple catalytic zones, mixtures of catalysts and inert supports used, volumetric concentration gradient of catalyst along a catalyst fixed bed or zone thereof, etc.), wherein weight fractions of the catalysts in a particular zone are correlated with the temperature of that particular zone to contribute to the averaging of the temperatures to yield the WABT. Without wishing to be limited by theory, a temperature across the catalyst bed is expected to increase along a length of the catalyst bed, in the direction of the flow through the reactor. As will be appreciated by one of skill in the art, and with the help of this disclosure, the reaction between hydrogen sulfide and ethylene oxide to produce BME is exothermic, and since the reaction occurs primarily in the catalyst bed, the temperature of the catalyst bed (e.g., WABT) will be higher at an exit point than at an entry point. Further, as the temperature reaches a boiling point of hydrogen sulfide at the pressure inside the reactor, a portion of the hydrogen sulfide will convert to a gas phase and absorb some of the heat of reaction, thereby preventing a temperature inside the catalyst bed from getting too high and potentially damaging the catalyst. Without wanting to be limited by theory, it is believed that the enthalpy generated during the exothermic reaction of H₂S with ethylene oxide is sufficient to provide the latent heat of vaporization necessary to vaporize some, most, or all of the liquid H₂S after it enters the reactor. In one or more embodiments disclosed herein, the heat of vaporization of H₂S is about 0.11 kcal/gm (about 198 BTU/lb) or about 3.7 kcal/mole (about 6,670 BTU/lbmole).

In an embodiment, a process for producing BME can be performed at a pressure (e.g., the reactor can be characterized by a pressure) in a range of from 2068 kPa (300 psig) to 3447 kPa (500 psig), alternatively from 2413 kPa (350 psig) to 3275 kPa (475 psig), or alternatively from 2758 kPa (400 psig) to 3103 kPa (450 psig). In some embodiments, a process for producing BME can be performed at a pressure of 3103 kPa (450 psig). According to the disclosed embodiments, a pressure in the reactor has to be high enough such that the hydrogen sulfide is a liquid at the temperature inside the reactor or catalyst bed, and at the same time the pressure cannot be too high as to prevent hydrogen sulfide vaporization when the temperature increases.

In an embodiment, a weight hourly space velocity (WHSV) of the ethylene oxide can be in a range of from 0.1 to 1, alternatively from 0.2 to 0.8, or alternatively from 0.4 to 0.6. Generally, the weight hourly space velocity refers to a mass of reagents fed per hour divided by a mass of catalyst used in a particular reactor. The units for WHSV are (mass reagent)/(mass catalyst - hr), expressed and reported as hr⁻¹.

In an embodiment, a process for producing BME can further comprise converting, in the reactor, at least a portion of the hydrogen sulfide from a liquid phase to a vapor phase to absorb a heat of reaction created in the step of reacting. The reaction of ethylene oxide with hydrogen sulfide to produce BME is exothermic, and since in an embodiment the reactor does not employ an external cooling fluid or internal heat exchange elements or devices, converting at least a portion of the hydrogen sulfide from a liquid phase to a vapor phase allows for temperature control inside the reactor (e.g., adiabatic reactor). As previously described, the heat of reaction generated from the reaction with H₂S and ethylene oxide is sufficient to provide the latent heat of vaporization necessary for the H₂S in the reactor to transition from the liquid state into vapor. Without wishing to be limited by theory, for the reactor to operate adiabatically, or near adiabatically, the heat of reaction generated is essentially equal to the latent heat of vaporization of H₂S. Therefore the total enthalpy in the reactor is zero or close to zero, allowing the equilibrium between the heat of reaction and heat of vaporization to essentially control the temperature within the reactor.

In an embodiment, the step of reacting ethylene oxide with hydrogen sulfide, and the step of converting at least a portion of the hydrogen sulfide from a liquid phase to a vapor phase can occur simultaneously.

In some embodiments, a process for producing BME can further comprise recovering a vapor phase of the hydrogen sulfide from the reactor; condensing at least a portion of the vapor phase of the hydrogen sulfide to a liquid phase; and recycling at least a portion of the liquid phase of the hydrogen sulfide to the reactor (e.g., to the hydrogen sulfide stream).

In an embodiment, an effluent stream can flow out of the reactor, wherein the effluent stream can comprise a reaction product comprising BME that is produced in the reactor by a reaction of hydrogen sulfide and ethylene oxide in liquid phase in presence of the solid catalyst (e.g., heterogeneous catalysis). For purposes of the disclosure herein, the term "reaction product" refers to any reaction products that are produced by the reaction between hydrogen sulfide and ethylene oxide, including primarily BME, and any unreacted ethylene oxide. Further, for purposes of the disclosure herein, the term "reaction product" is defined on a hydrogen sulfide-free basis, e.g., the reaction product excludes hydrogen sulfide.

In an embodiment, the effluent stream can comprise the reaction product and hydrogen sulfide. In such embodiment, the effluent stream can be subjected to a separation step to separate the hydrogen sulfide from the reaction product. For example, the effluent stream can be introduced to a high pressure stripping column wherein the hydrogen sulfide is vaporized and recovered as hydrogen sulfide vapor phase (overhead stream), which can be subsequently condensed and recycled back to the reactor. The reaction product can be recovered as a bottoms stream from the high pressure stripping column.

In an embodiment, the reaction product in the effluent can consist essentially of the BME and the TDG (e.g., the reaction product can comprise less than 1 wt.%, less than 0.1 wt.%, less than 0.01 wt.%, or less than 0.001 wt.% ethylene oxide, based on a total weight of the reaction product). In an embodiment, an effluent of the reactor (e.g., effluent stream) can comprise the reaction product, wherein the reaction product in the effluent further comprises equal to or greater than 99 wt.%, alternatively equal to or greater than 99.5 wt.%, or alternatively equal to or greater than 99.9 wt.% BME (with any remainder comprising TDG and/or unreacted ethylene oxide). According to the embodiments of this disclosure, it is possible to produce a reaction product having upwards of 99 wt.% purity BME

In an embodiment, an effluent of the reactor (e.g., effluent stream) can comprise the reaction product, wherein the reaction product in the effluent further comprises less than 0.5 wt.%, alternatively less than 0.25 wt.%, alternatively less than 0.1 wt.%, alternatively less than 0.05 wt.%, or alternatively less than 0.01 wt.% TDG and/or unreacted ethylene oxide. TDG can form by exothermic reaction of BME with ethylene oxide. As will be appreciated by one of skill in the art, and with the help of this disclosure, TDG is generally present as an undesirable product (e.g., impurity) in reaction products from BME production processes. Without wishing to be limited by theory, the heterogeneous ion exchange resin as disclosed herein catalyzes selectively the formation of BME from ethylene oxide and hydrogen sulfide, and as such only a very low amount of undesirable products such as TDG is formed, or no amount of undesirable products such as TDG is formed.

In some embodiments, the reaction product is essentially free of TDG, e.g., the reaction product comprises no TDG. In an embodiment, the reaction product consists of, or consists essentially of, BME. In an embodiment, no TDG can be detected (e.g., by gas chromatography) in the reaction product. In an embodiment, the amount of TDG in the reaction product is 0 wt.% or 0 mol%.

In some embodiments, the reaction product is essentially free of unreacted ethylene oxide, e.g., the reaction product comprises no unreacted ethylene oxide. In an embodiment, the amount of unreacted ethylene oxide in the reaction product is 0 wt.% or 0 mol%.

In an embodiment, no detectable amount of EDT is present in the reaction product when analyzing a sample of an effluent of the reactor via gas chromatography to two decimal places for weight percent or to three decimal places for mole percent. Conventional processes for the production of BME from the reaction of ethylene oxide with hydrogen sulfide generally lead to the production of EDT, which is another undesirable product (e.g., impurity) in the reaction products, in addition to TDG.

In some embodiments, the reaction product is essentially free of EDT, e.g., the reaction product comprises no EDT. In an embodiment, the amount of EDT in the reaction product is 0.00 wt.% or 0.000 mol%.

Measurements can be taken with any gas chromatography technique known by those skilled in the art with the aid of this disclosure. Examples of suitable gas chromatography (GC) techniques include those which utilize a capillary column having a nonpolar stationary phase (e.g., 100% dimethylpolysiloxane). Using a capillary column as described allows for separation by component volatility (i.e., boiling point) only. Nonlimiting examples of capillary columns are VF-1ms and CP-Sil 5 CB. The detector used in the GC technique may be a TCD detector or FID detector. A TCD detector can detect water and H₂S in a single analysis, while FID can detect the reaction products, for example, in high purity.

For purposes of the disclosure herein, a selectivity to BME can be calculated by dividing an amount of ethylene oxide that was converted to BME in a given time period in a flow reactor by the total amount of ethylene oxide that was converted into any reaction product, including BME, over the exact same time period in the same flow reactor. In an embodiment, a process for producing BME can be characterized by a selectivity to BME of greater than 99 wt.%, alternatively greater than 99.5 wt.%, or alternatively greater than 99.9 wt.%, based on a total weight of ethylene oxide that was converted to BME divided by a total weight of ethylene oxide that was converted into the reaction product. In an embodiment, the solid catalyst having weakly basic active groups can allow a conversion of the ethylene oxide with a selectivity to BME of greater than 99 wt.%, based on a total weight of ethylene oxide that was converted to BME divided by a total weight of ethylene oxide that was converted into the reaction product. Without wishing to be limited by theory, the heterogeneous ion exchange resin having weakly basic active groups could selectively enable the formation of BME, while selectively inhibiting the formation of undesirable products such as EDT and TDG.

For purposes of the disclosure herein, a conversion of ethylene oxide to reaction products can be calculated by dividing an amount of ethylene oxide that was converted to reaction product in a given time period in a flow reactor by the total amount of ethylene oxide that was introduced to the same flow reactor, over the exact same time period. In an embodiment, the step of reacting ethylene oxide with hydrogen sulfide to produce BME can include a conversion of ethylene oxide to the reaction product that is greater than 99 wt.%, alternatively greater than 99.5 wt.%, or alternatively greater than 99.9 wt.%, based on the weight of ethylene oxide that was converted to reaction product divided by the weight of ethylene oxide fed to the reactor.

The present invention provides a process for producing BME can comprise reacting, in an adiabatic continuous flow reactor having a fixed bed containing a solid catalyst comprising a heterogeneous ion exchange resin, hydrogen sulfide and ethylene oxide in the presence of the solid catalyst to yield a reaction product comprising BME; wherein during steady state operation of the reactor, the hydrogen sulfide and the ethylene oxide can be present in a mole ratio in a range of 9:1 to 20:1. Wherein the process can be performed at a temperature in a range of from 30 °C to 80 °C, and a pressure of 3103 kPa (450 psig); wherein the heterogeneous ion exchange resin comprises a styrene-DVB copolymer with weakly basic active groups comprising alkyl amines; and wherein a selectivity to BME can be greater than 99 wt.%, based on a total weight of ethylene oxide that was converted to BME divided by a total weight of ethylene oxide that was converted into the reaction product.

Also disclosed, but not part of the presently claimed invention, is a reactor system for producing BME can comprise an adiabatic continuous flow reactor; an ethylene oxide stream feeding ethylene oxide to the reactor; a hydrogen sulfide stream feeding hydrogen sulfide to the reactor; a fixed bed containing a solid catalyst placed in the reactor, wherein the solid catalyst comprises a styrene-DVB copolymer with weakly basic active groups comprising alkyl amines; and an effluent stream flowing from the reactor, wherein the effluent stream comprises a reaction product comprising BME produced in the reactor by a reaction of hydrogen sulfide and ethylene oxide in presence of the solid catalyst; wherein the hydrogen sulfide and the ethylene oxide are present in the reactor during steady state operation in a mole ratio ranging from 9:1 to 20:1; wherein the reactor operates at a temperature in a range of from 30 °C to 80 °C and a pressure of 3103 kPa (450 psig); wherein a conversion of ethylene oxide to the reaction product can be greater than 99 wt.%, based on the weight of ethylene oxide that was converted to reaction product divided by the weight of ethylene oxide fed to the reactor; and wherein a selectivity to BME can be greater than 99 wt.%, based on a total weight of ethylene oxide that was converted to BME divided by a total weight of ethylene oxide that was converted into the reaction product.

In an embodiment, a process for producing BME as disclosed herein can advantageously display improvements in one or more process characteristics when compared to conventional processes for the production of BME. In conventional processes for the production of BME, generally, continuous stirred-tank reactors are employed. In an embodiment, a PFR as disclosed herein can advantageously allow for safer operation. In an embodiment, a process for producing BME as disclosed herein can advantageously employ an adiabatic reactor (e.g., an adiabatic PFR), which is easier to operate due to no secondary heat exchange requirement (e.g., in the case of an adiabatic reactor, there is no need to cool the reactor by using energy input from outside the reactor). In an embodiment, a process for producing BME as disclosed herein can advantageously employ an adiabatic reactor (e.g., an adiabatic PFR), which is smaller (i.e., requires a smaller footprint) than a CSTR to achieve the same conversion. In an embodiment, a process for producing BME as disclosed herein can advantageously employ an adiabatic reactor (e.g., an adiabatic PFR), which has lower capital and operating costs compared to a comparable CSTR reactor.

In an embodiment, a process for producing BME as disclosed herein can advantageously allow for an increased selectivity of ethylene oxide to BME (of 99%), when compared to other conventional processes for the production of BME (typically 92%). In an embodiment, a process for producing BME as disclosed herein can advantageously allow for near quantitative conversion of ethylene oxide to reaction products (of 99%) when compared a conventional processes for the production of BME

In an embodiment, a process for producing BME as disclosed herein can advantageously employ a solid catalyst (as opposed to a liquid catalyst or liquid initiator used in conventional processes for the production of BME), and as such the catalyst remains in the reactor and is not removed with the products, as is the case with liquid catalysts and initiators, which would further require an additional separation step to recover the products from the liquid catalysts and/or initiators.

In an embodiment, a process for producing BME as disclosed herein can advantageously eliminate the need for using a liquid caustic initiator for the reaction between ethylene oxide and hydrogen sulfide by using a solid basic catalyst.

In an embodiment, a process for producing BME as disclosed herein can advantageously allow for a higher rate of production (e.g., higher BME output) owing to a higher heat exchanging capacity of the reactor.

In an embodiment, a process for producing BME as disclosed herein can advantageously allow for producing a significantly lower amount of BME impurities (less than about 0.5 wt.%), such as TDG, EDT, or both, when compared to other conventional processes for the production of BME, which can produce 5-7 wt.% impurities that have to be separated and disposed of. As will be appreciated by one of skill in the art, and with the help of this disclosure, EDT has an unpleasant odor, and as such there are certain difficulties associated with EDT separation and disposal. Additional advantages of the systems and/or methods for the production of a BME as disclosed herein can be apparent to one of skill in the art viewing this disclosure and include but are not limited to a smaller reactor size, a smaller reactor footprint, lower capital costs, and lower operating costs.

### EXAMPLES

The subject matter having been generally described, the following examples are given as particular embodiments of the disclosure and to demonstrate the practice and advantages thereof. It is understood that the examples are given by way of illustration and are not intended to limit the specification of the claims to follow in any manner.

The following general experimental procedure was used for all examples, unless otherwise indicated.

Producing beta-mercaptoethanol (BME) was studied for various experimental conditions, such as catalyst, temperature, and reactant ratios. More specifically, the synthesis of BME was investigated for two different catalysts, AMBERLYST® A21 resin and LEWATIT® MP 62 resin.

The BME synthesis reaction from ethylene oxide and H₂S is highly exothermic: C₂H₄O (EO) + H₂S → C₂H₆SO (BME) ΔHᵣₓₙ = -75,575 BTU/lbmole C₂H₆SO (BME) + C₂H₄O (EO) → (C₂H₅OH)₂S (TDG) ΔHᵣₓₙ = -75,870 BTU/lbmole

BME was produced in a fixed bed catalytic flow reactor operating in downflow. The reactor was a 1 inch inner diameter stainless steel jacketed reactor. The catalyst was either AMBERLYST® A21 resin or LEWATIT® MP 62 resin. Temperatures were controlled using a 50/50 glycol/water mixture flowing in upflow through the jacket. The glycol/water mixture temperature was controlled using an external Lauda RK20 constant temperature bath (of approximately 5 gallons).

During adiabatic experiments with the LEWATIT® MP 62 resin catalyst, there was no temperature control. The jacket temperature was raised sufficiently to initiate reaction, and then the flow of heat transfer fluid to the external jacket was stopped.

The catalyst packing had three equal zones (by volume): a top zone, a middle zone, and a bottom zone. The top zone was 2/3 chemically inert solid diluent (ALUNDUM® Alumina) and one-third catalyst by volume. The middle zone was 1/2 catalyst and 1/2 inert solid diluent (ALUNDUM® Alumina), again by volume. The bottom zone was all catalyst.

The ethylene oxide and hydrogen sulfide (H₂S) were fed independently, and each flowrate was controlled using a Brooks mass flow controller. The two feeds were combined prior to entering the reactor and mixed in a static mixer. Static mixing was achieved by passing the feed through 2 inches of glass beads. There was a thermocouple in the reactor inlet (5 inches above the catalyst bed) just downstream of the static mixer. The temperature measured by this thermocouple stayed at ambient temperature throughout all of the experiments.

Product analysis was conducted using a HP-6890 gas chromatograph (GC) equipped with a thermal conductivity detector. This method allows for quantification of crude BME, water, and H₂S all in one analysis. On-line product samples were routed directly to the GC from the reactor effluent. The GC was equipped with a 30 m x 0.32 mm 4.0 µm film CP-Sil CB column. The initial temperature was 35 °C with a 5 min hold time and was ramped to a final temperature of 260 °C at a rate of 10 °C/min with a 10 min hold time. The carrier gas was helium at a flow rate of 1.0 mL/min. The thermal conductivity (TCD) response factors used were as follows: ethylene oxide 0.58, H₂S 0.89, BME 0.73, thiodiglycol (TDG) 0.75, H₂O 0.55, and ethanedithiol (EDT) 0.75.

The catalysts as received were 50 wt.% water. The AMBERLYST® A21 resin catalyst was dried using flowing methanol at 40 °C. However, this procedure resulted simply in displacement of water with methanol to a large extent. Thus, there was methanol in the effluent from the reactors for most of the course of the experimental work, and some of this methanol was still present in the LEWATIT® MP 62 resin experiments. The LEWATIT® MP 62 resin was dried using flowing nitrogen at 55 °C. This drying procedure was not hot enough to remove all of the water from the catalyst, so there was a considerable amount of water in the reactor effluent at the start of the LEWATIT® MP 62 resin experiments.

### EXAMPLE 1

The reaction of ethylene oxide with hydrogen sulfide in the presence of AMBERLYST® A21 resin catalyst was investigated for various hydrogen sulfide (H₂S) to ethylene oxide (EO) molar ratios, and the experimental conditions are displayed in Table 1.

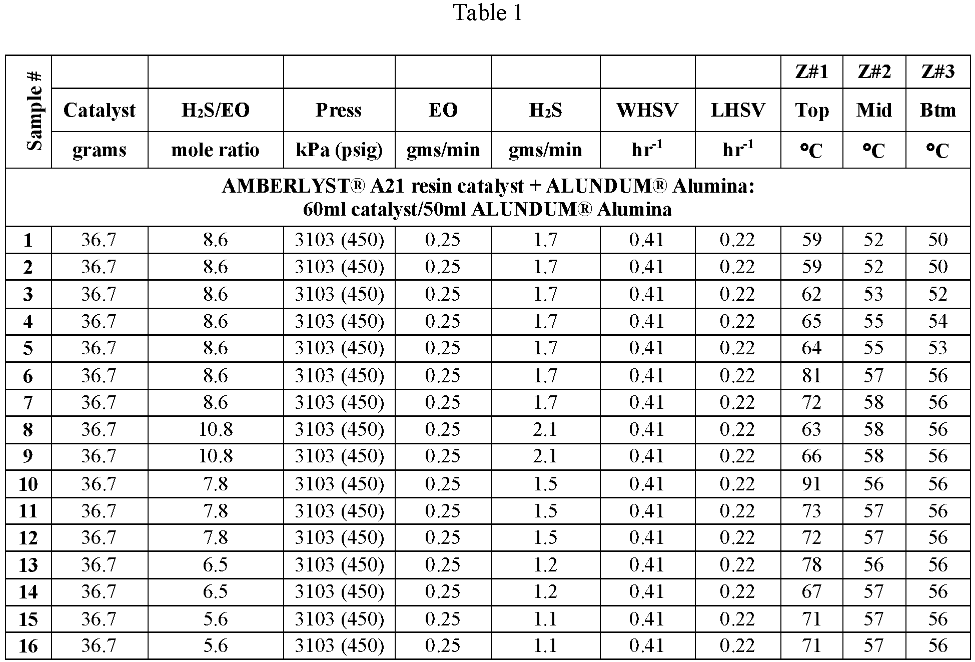

Data were collected first as rate vs. temperature at fixed pressure and fixed hydrogen sulfide to ethylene oxide molar ratios (for samples 1 through 7) and then at fixed temperature (56 °C outlet) and varying H₂S:EO mole ratios (for samples 8 through 16). The space velocity was calculated based upon the EO feed rate. The results for samples 1 through 16 were analyzed as previously described, and the resulting data based on wt.% are displayed in Table 2 and the resulting data based on mol% are displayed in Table 3.

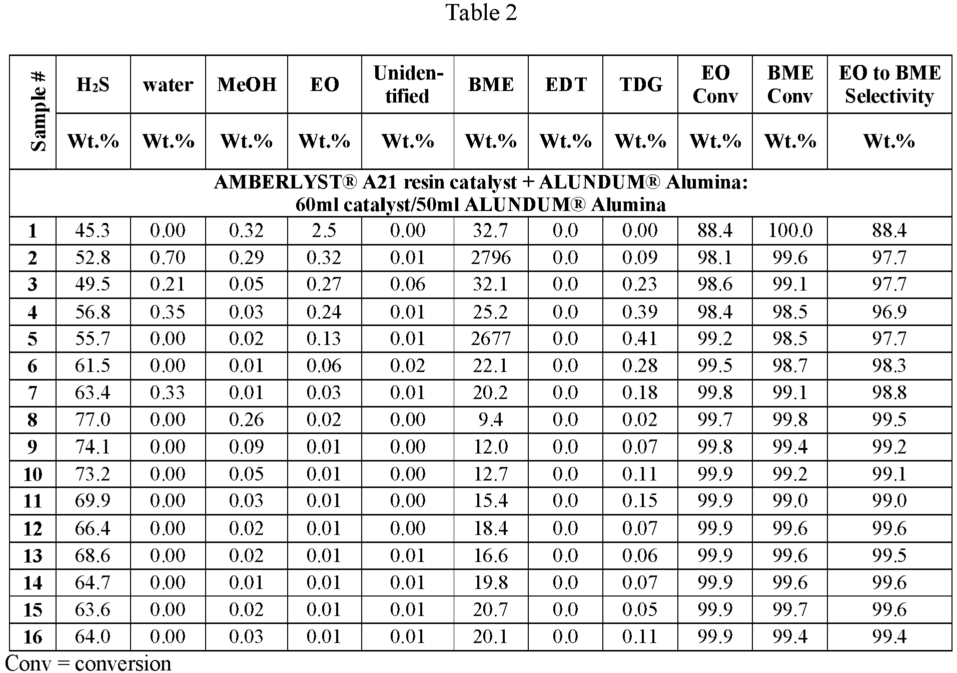

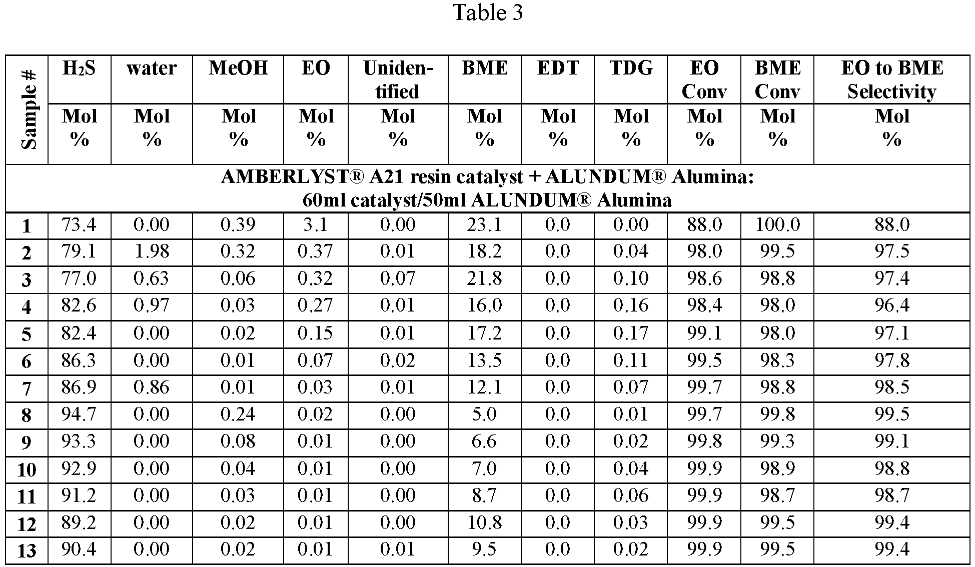

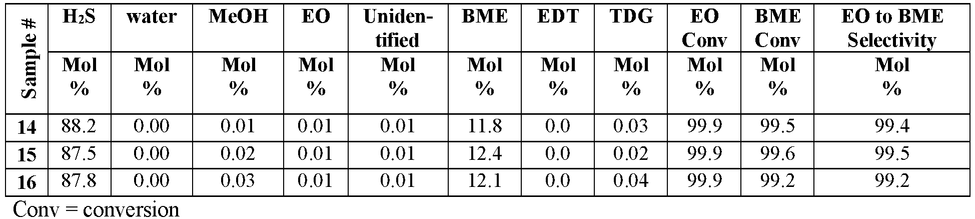

### EXAMPLE 2

The reaction of ethylene oxide with hydrogen sulfide in the presence of LEWATIT® MP 62 resin catalyst was investigated for various hydrogen sulfide to ethylene oxide molar ratios, and the experimental conditions are displayed in Table 4. The reactor was operated with external cooling (analogous to the data in Example 1).

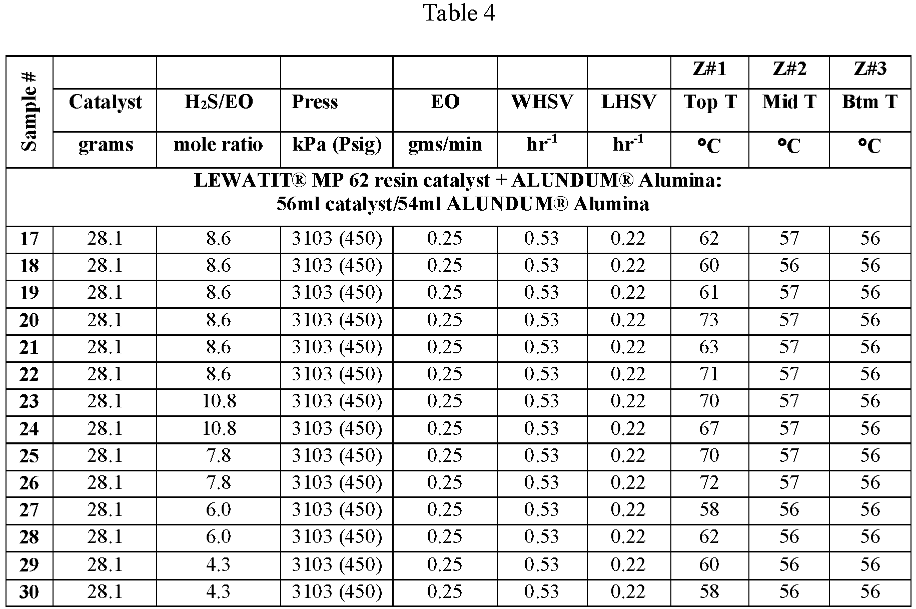

Data were collected first as rate vs. temperature at fixed pressure and fixed hydrogen sulfide to ethylene oxide molar ratios (for samples 17 through 22) and then at fixed temperature (56 °C outlet) and varying H₂S:EO mole ratios (for samples 23 through 30). The space velocity was calculated based upon the EO feed rate. The results for samples 17 through 30 were analyzed as previously described, and the resulting data based on wt.% are displayed in Table 5 and the resulting data based on mol% are displayed in Table 6.

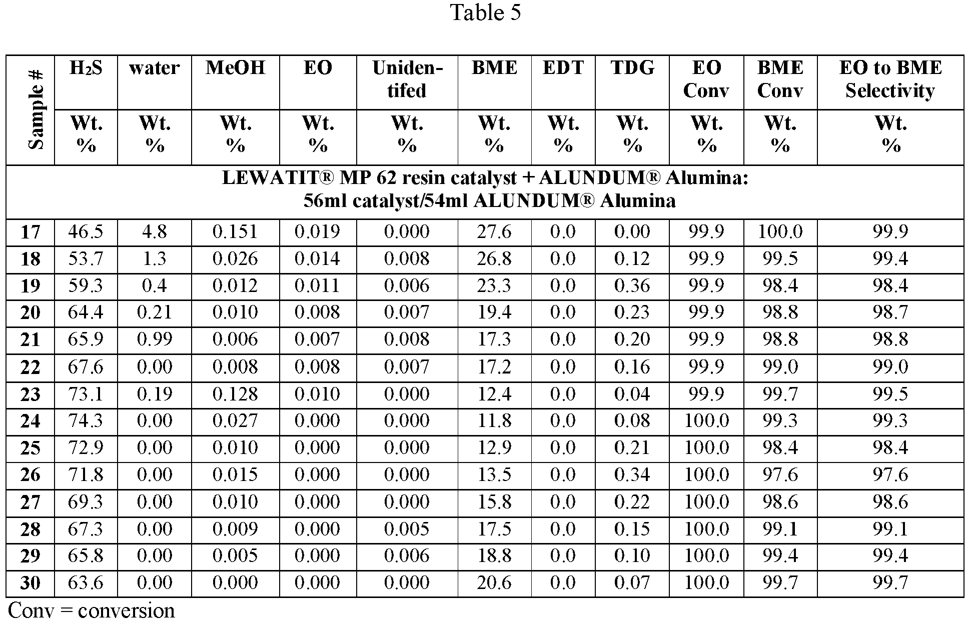

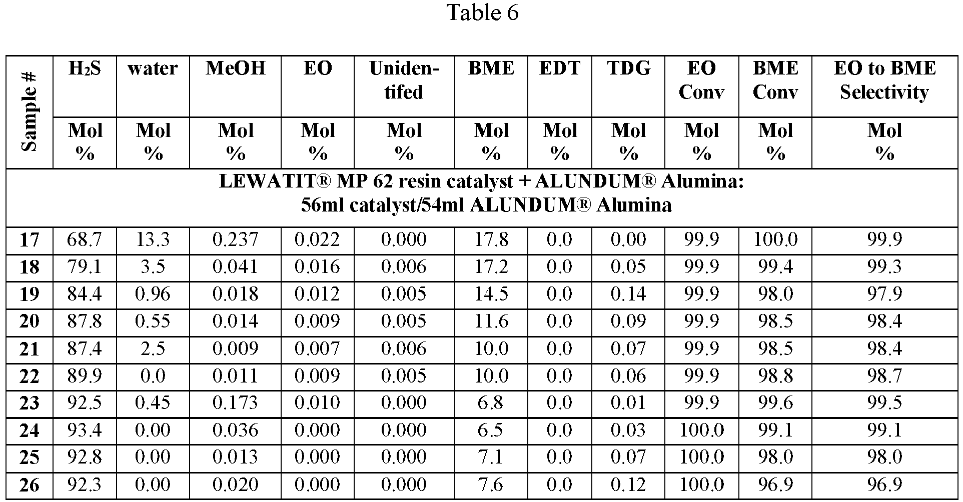

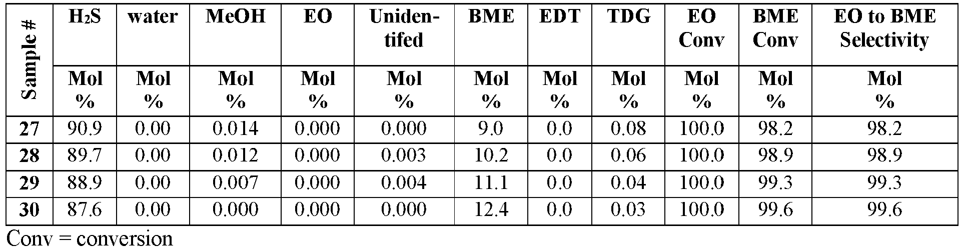

### EXAMPLE 3

The reaction of ethylene oxide with hydrogen sulfide in the presence of LEWATIT® MP 62 resin catalyst was investigated for various hydrogen sulfide to ethylene oxide molar ratios, and the experimental conditions are displayed in Table 7. The reactor was operated adiabatically, with no external heat transfer.

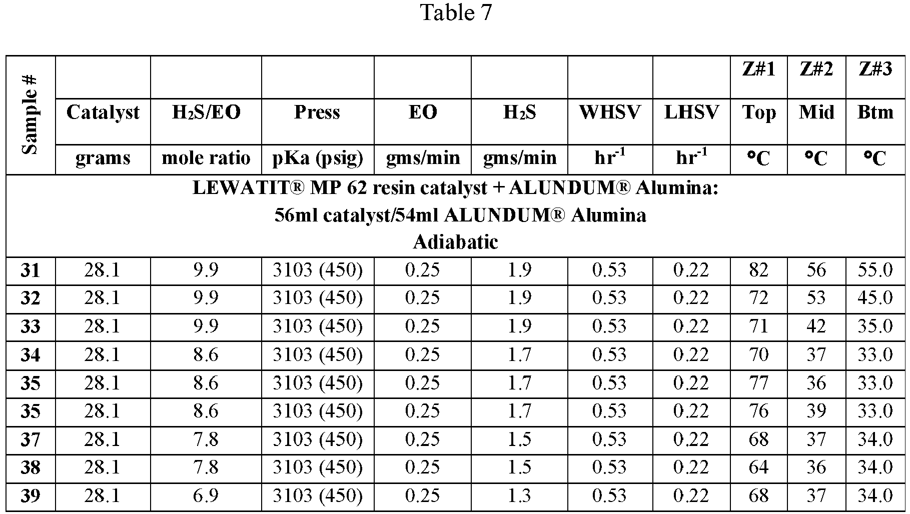

Data were collected first as rate vs. temperature at fixed pressure and fixed hydrogen sulfide to ethylene oxide molar ratios (for samples 17 through 22) and then at fixed temperature (56 °C outlet) and varying H₂S:EO mole ratios (for samples 23 through 30). The space velocity was calculated based upon the EO feed rate. The results for samples 31 through 39 were analyzed as previously described, and the resulting data based on wt.% are displayed in Table 8 and the resulting data based on mol% are displayed in Table 9.

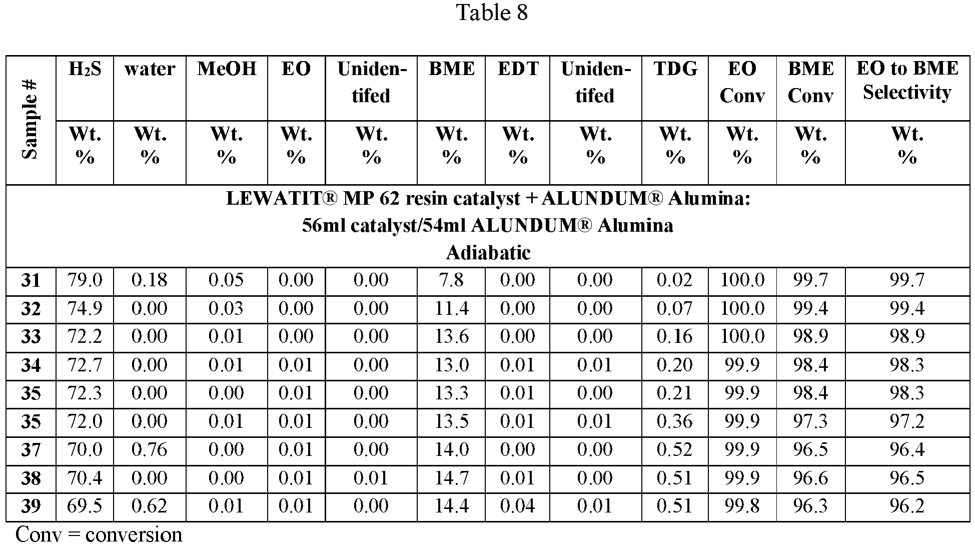

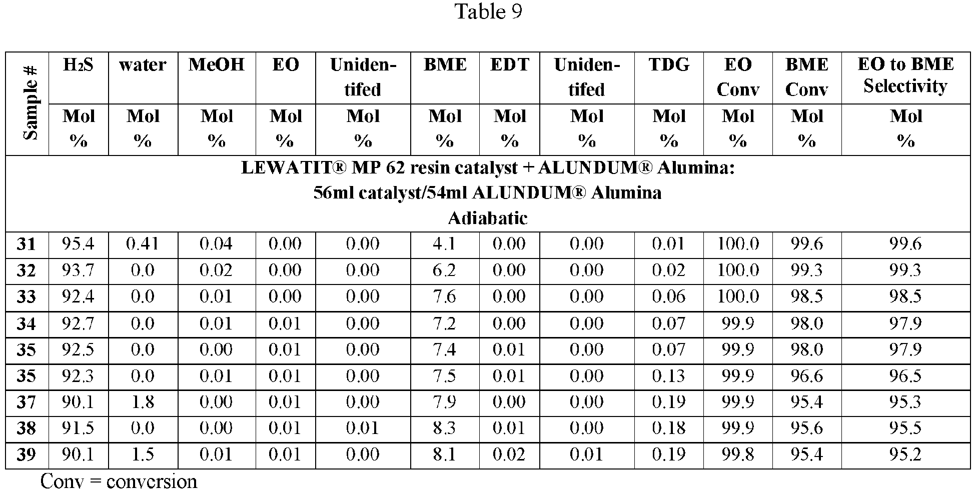

Referring to the data presented in the Examples 2 and 3, the results of running the BME synthesis reaction in the presence of the two different catalysts and under different conditions is now discussed herein.

Catalyst Packing and Preparation. Both AMBERLYST® A21 resin and LEWATIT® MP 62 resin worked well for the BME synthesis. The catalyst packing with ALUNDUM® Alumina diluent was found to perform in a satisfactory manner.

The catalyst should be dried prior to first usage. The catalyst (as received) can be as much as 50 wt.% water. In addition, ion exchange catalysts should not be exposed to prolonged temperatures in excess of 80 °C, as the catalysts can lose activity. Completely anhydrous conditions prior to start-up cannot be achieved by simply sparging with heated nitrogen. Heating catalysts in flowing nitrogen for 20-24 hours at 60-65 °C could help condition catalysts prior to use in the reactor for BME synthesis. Moisture removal using flowing alcohol (methanol or isopropanol) leaves undesirable residual alcohols. Should the nitrogen drying procedure not remove all the moisture, there could be some moisture present initially during start-up; however, such moisture would be preferable to residual alcohols.

Process Conditions for BME Synthesis from Ethylene Oxide Using Basic Catalysts. The conditions that appeared most favorable were: weight average bed temperature (WAT) of 65-70 °C; pressure of at least 3103 kPa (450 psig), ethylene oxide weight hourly space velocity of 0.5 to 0.6, and H₂S/EO mole ratio of 9:1 (or greater). H₂S/EO mole ratios higher than 9:1 resulted in less heavies (EDT and TDG) production. Pressure and space velocities were not varied during the experiments.

Product Composition and Ethylene Oxide Conversion. Ethylene oxide conversion was essentially 100%. Virtually all the EO was consumed during the reaction. Products of reaction (at 9:1 or higher H₂S/EO mole ratios) were BME and TDG only. By comparison with conventional BME synthesis processes that could yield a heavies production of 5-7 wt.%, the data in Examples 2 and 3 indicate that for 9:1 or higher H₂S/EO mole ratios, heavies production was well below 0.5 wt.%.

Effect of H₂S/EO Mole Ratio. Conversion of EO was quantitative at all mole ratios that were tested. Selectivity to BME was greater than 99% for H₂S/EO mole ratios in excess of 9:1. There was measurable EDT formation at mole ratios of 8:1 or less. Two additional unidentified products were also detected at mole ratios of less than 8:1 during the adiabatic experiments (Example 3). There was water in the reactor effluent concurrent with the detection of EDT, as expected (Sample 39 in Example 3).

Unidentified Peaks. While no efforts were made to identify the minor components produced at lower H₂S/EO mole ratios, possibilities include ethylene sulfide and its derivatives. In addition, since there was always some methanol in the feedstock, some of the unidentified peaks could have been products of reaction of methanol. At H₂S/EO mole ratios of greater than 8:1, these unidentified peaks were not present.

Utility for BME Synthesis. The selectivity of EO to BME was improved from 92% for conventional BME synthesis processes to >99% for 9:1 or higher H₂S/EO mole ratios. As a consequence of this improved selectivity, the H₂S usage needed for BME synthesis could be reduced by 5-7%, when compared to conventional BME synthesis processes. Also, heavies production could be curtailed by more than 90%, when compared to conventional BME synthesis processes.

### EXAMPLE 4

The activation energy for BME synthesis from ethylene oxide and hydrogen sulfide was also investigated in the presence of the LEWATIT® MP 62 resin catalyst. The activation energy for BME synthesis on LEWATIT® MP 62 resin catalyst was found to be extremely low.

Figure 1 displays an Arrhenius plot of activation energies for LEWATIT® MP-62 catalyzed BME synthesis from ethylene oxide and H₂S. The Arrhenius plot of Figure 1 is a plot of log mole% BME vs. inverse temperature. Arrhenius rate expressions for BME synthesis and related reactions are shown in Equations 1A, 1B and 1C: $log mole % EO = - 2.783 - 1245.4 ∗ 1 / T \left(1/R\right) ; {R}^{2} = 0.01$ (1A) $log mole % BME = - 0.718 + 1710.8 ∗ 1 / T \left(1/R\right) ; {R}^{2} = 0.551$ (1B) $log mole % TDG = - 22.682 + 12753.6 ∗ 1 / T \left(1/R\right) ; {R}^{2} = 0.424$ (1C)

From the R squared value in Equation 1A, it can be readily inferred that temperature has almost no effect on EO conversion, with temperatures above 50 °C resulting in essentially 100% EO conversion.

While preferred embodiments of the invention have been shown and described, modifications thereof can be made by one skilled in the art without departing from the teachings of the invention. The embodiments described herein are exemplary only, and are not intended to be limiting. Many variations and modifications of the invention disclosed herein are possible and are within the scope of the invention. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). Use of the term "optionally" with respect to any element of a claim is intended to mean that the subject element is required, or alternatively, is not required. Both alternatives are intended to be within the scope of the claim. Use of broader terms such as comprises, includes, having, etc. should be understood to provide support for narrower terms such as consisting of, consisting essentially of, comprised substantially of, etc.

Accordingly, the scope of protection is not limited by the description set out above but is only limited by the claims which follow, that scope including all equivalents of the subject matter of the claims. Each and every claim is incorporated into the specification as an embodiment of the present invention. Thus, the claims are a further description and are an addition to the preferred embodiments of the present invention. The discussion of a reference in the disclosure is not an admission that it is prior art to the present invention, especially any reference that may have a publication date after the priority date of this application.

## Claims

1. A process comprising:
reacting, in a reactor having a fixed bed containing a solid catalyst comprising a heterogeneous ion exchange resin, hydrogen sulfide and ethylene oxide in the presence of the solid catalyst to yield a reaction product comprising beta-mercaptoethanol, wherein during steady state operation of the reactor, the hydrogen sulfide and the ethylene oxide are present in a mole ratio in a range of 9:1 to 20:1.

2. The process of claim 1, further comprising:
converting, in the reactor, at least a portion of the hydrogen sulfide from a liquid phase to a vapor phase to absorb a heat of reaction created in the step of reacting.

3. The process of claim 2, wherein the step of reacting and the step of converting occur simultaneously.

4. The process of any one of claims 1 to 3, wherein no internal and/or external cooling source is used to cool the reactor.

5. The process of any one of claims 1 to 4, wherein an effluent of the reactor comprises the reaction product, wherein the reaction product in the effluent further comprises less than 0.5 wt.% thiodiglycol.

6. The process of claim 5, wherein the reaction product in the effluent consists essentially of the beta-mercaptoethanol and the thiodiglycol.

7. The process of any one of claims 1 to 6, wherein no detectable amount of ethanedithiol is present in the reaction product when analyzing a sample of an effluent of the reactor via gas chromatography to two decimal places for weight percent or to three decimal places for mole percent.

8. The process of any one of claims 1 to 7, wherein the reactor is an adiabatic reactor.

9. The process of any one of claims 1 to 8, wherein the solid catalyst has weakly basic active groups to allow a conversion of the ethylene oxide by the hydrogen sulfide with a selectivity to beta-mercaptoethanol greater than 99 wt.% based on a total weight of the ethylene oxide that converts to beta-mercaptoethanol divided by a total weight of the ethylene oxide that converts into the reaction product.

10. The process of any one of claims 1 to 9, wherein the solid catalyst comprises a styrenedivinylbenzene copolymer with weakly basic active groups comprising alkyl amines.

11. The process of any one of claims 1 to 10, wherein the step of reacting includes a conversion of ethylene oxide to the reaction product which is greater than 99 wt.% based on a weight of the ethylene oxide that converts to the reaction product divided by a weight of the ethylene oxide fed to the reactor.

12. The process of any one of claims 1 to 11, performed at a temperature in a range of 30 °C to 80 °C.

13. The process of any one of claims 1 to 12, wherein the fixed bed of the reactor has a weight average bed temperature of 65 °C to 70 °C.

14. The process of any one of claims 1 to 13, wherein a weight hourly space velocity of the ethylene oxide is in a range of 0.1 to 1 hr⁻¹.

15. The process of any one of claims 1 to 14, performed at a pressure in a range of 2068 kPa (300 psig) to 3447 kPa (500 psig).

16. The process of any one of claims 1 to 15, performed at a pressure of 3103 kPa (450 psig).

17. The process of any one of claims 1 to 16, further comprising:
recovering a vapor phase of the hydrogen sulfide from the reactor;
condensing the vapor phase of the hydrogen sulfide to a liquid phase; and
recycling the liquid phase of the hydrogen sulfide to the reactor.

## Patentansprüche

1. Vorgang, umfassend:
Reagieren, in einem Reaktor, der ein Festbett aufweist, der einen festen Katalysator enthält, der ein heterogenes lonenaustauscherharz, Schwefelwasserstoff und Ethylenoxid umfasst, in Gegenwart des festen Katalysators, um ein Reaktionserzeugnis zu ergeben, das ein Beta-Mercaptoethanol umfasst, wobei während eines stationären Betriebs des Reaktors der Schwefelwasserstoff und das Ethylenoxid in einem Molverhältnis in einem Bereich von etwa 9:1 bis etwa 20:1 vorliegen.

2. Vorgang nach Anspruch 1, ferner umfassend:
Umsetzen, in dem Reaktor, wenigstens eines Anteils des Schwefelwasserstoffs von einer flüssigen Phase in eine Dampfphase, um eine Reaktionswärme zu absorbieren, die in dem Reaktionsschritt erzeugt wird.

3. Vorgang nach Anspruch 2, wobei der Reaktionsschritt und der Umsetzungsschritt etwa gleichzeitig ablaufen.

4. Vorgang nach einem der Ansprüche 1 bis 3, wobei keine interne und/oder externe Kühlquelle verwendet wird, um den Reaktor abzukühlen.

5. Vorgang nach einem der Ansprüche 1 bis 4, wobei ein Ausfluss des Reaktors das Reaktionserzeugnis umfasst, wobei das Reaktionserzeugnis in dem Ausfluss ferner weniger als etwa 0,5 Gew.-% Thiodiglykol umfasst.

6. Vorgang nach Anspruch 5, wobei das Reaktionserzeugnis in dem Ausfluss im Wesentlichen aus dem Beta-Mercaptoethanol und dem Thiodiglykol besteht.

7. Vorgang nach einem der Ansprüche 1 bis 6, wobei keine nachweisbare Menge Ethandithiol in dem Reaktionserzeugnis vorhanden ist, wenn eine Probe eines Ausflusses des Reaktors über Gaschromatographie auf zwei Dezimalstellen für Gewichtsprozent oder auf drei Dezimalstellen für Molprozent analysiert wird.

8. Vorgang nach einem der Ansprüche 1 bis 7, wobei der Reaktor ein adiabatischer Reaktor ist.

9. Vorgang nach einem der Ansprüche 1 bis 8, wobei der feste Katalysator schwach basische aktive Gruppen aufweist, um eine Umsetzung des Ethylenoxids durch den Schwefelwasserstoff mit einer Selektivität zu Beta-Mercaptoethanol von mehr als etwa 99 Gew.-% zu ermöglichen, basierend auf einem Gesamtgewicht des Ethylenoxids, das sich in Beta-Mercaptoethanol umsetzt, geteilt durch das Gesamtgewicht des Ethylenoxids, das sich in das Reaktionserzeugnis umsetzt.

10. Vorgang nach einem der Ansprüche 1 bis 9, wobei der feste Katalysator ein Styrol-Divinylbenzol-Copolymer mit schwach basischen aktiven Gruppen umfasst, die Alkylamine umfassen.

11. Vorgang nach einem der Ansprüche 1 bis 10, wobei der Reaktionsschritt eine Umsetzung von Ethylenoxid in das Reaktionserzeugnis beinhaltet, das größer als etwa 99 Gew.-% ist, basierend auf einem Gewicht des Ethylenoxids, das sich in das Reaktionserzeugnis umsetzt, geteilt durch ein Gewicht des dem Reaktor zugeführten Ethylenoxids.

12. Vorgang nach einem der Ansprüche 1 bis 11, das bei einer Temperatur in einem Bereich von etwa 30 °C bis etwa 80 °C durchgeführt wird.

13. Vorgang nach einem der Ansprüche 1 bis 12, wobei das Festbett des Reaktors eine gewichtsmittlere Betttemperatur von etwa 65 °C bis etwa 70 °C aufweist.

14. Vorgang nach einem der Ansprüche 1 bis 13, wobei eine gewichtsstündliche Raumgeschwindigkeit des Ethylenoxids in einem Bereich von 0,1 bis 1 Stunde¹ liegt.

15. Vorgang nach einem der Ansprüche 1 bis 14, das bei einem Druck in einem Bereich von 2068 kPa (300 psig) bis 3447 kPa (500 psig) durchgeführt wird.

16. Vorgang nach einem der Ansprüche 1 bis 15, das bei einem Druck von 3103 kPa (450 psig) durchgeführt wird.

17. Vorgang nach einem der Ansprüche 1 bis 16, ferner umfassend:
Zurückgewinnen einer Dampfphase des Schwefelwasserstoffs aus dem Reaktor;
Kondensieren der Dampfphase des Schwefelwasserstoffs zu einer flüssigen Phase; und
Rückführen der flüssigen Phase des Schwefelwasserstoffs in den Reaktor.

## Revendications

1. Procédé comprenant :
la mise en réaction, dans un réacteur à lit fixe contenant un catalyseur solide comprenant une résine échangeuse d'ions hétérogène, du sulfure d'hydrogène et de l'oxyde d'éthylène en présence du catalyseur solide pour obtenir un produit de réaction comprenant du bêta-mercaptoéthanol, dans lequel, pendant le fonctionnement à l'état d'équilibre du réacteur, le sulfure d'hydrogène et l'oxyde d'éthylène sont présents dans un rapport molaire dans la plage d'environ 9 : 1 à environ 20 : 1.

2. Procédé selon la revendication 1, comprenant en outre :
la mise en conversion, dans le réacteur, d'au moins une partie du sulfure d'hydrogène d'une phase liquide en une phase vapeur pour absorber une chaleur de réaction créée lors de l'étape de mise en réaction.

3. Procédé selon la revendication 2, dans lequel l'étape de mise en réaction et l'étape de mise en conversion se déroulent à peu près simultanément.

4. Procédé selon l'une quelconque des revendications 1 à 3, dans lequel aucune source de refroidissement interne et/ou externe n'est utilisée pour refroidir le réacteur.

5. Procédé selon l'une quelconque des revendications 1 à 4, dans lequel un effluent du réacteur comprend le produit de réaction, dans lequel le produit de réaction dans l'effluent comprend en outre moins d'environ 0,5 % en poids de thiodiglycol.

6. Procédé selon la revendication 5, dans lequel le produit de réaction dans l'effluent est essentiellement constitué par le bêta-mercaptoéthanol et le thiodiglycol.

7. Procédé selon l'une quelconque des revendications 1 à 6, dans lequel aucune quantité détectable d'éthanedithiol n'est présente dans le produit de réaction lors de l'analyse d'un échantillon d'un effluent du réacteur par l'intermédiaire d'une chromatographie en phase gazeuse à deux décimales en pourcentage en poids ou à trois décimales pour cent molaire.

8. Procédé selon l'une quelconque des revendications 1 à 7, dans lequel le réacteur est un réacteur adiabatique.

9. Procédé selon l'une quelconque des revendications 1 à 8, dans lequel le catalyseur solide a des groupes actifs faiblement basiques pour permettre une conversion de l'oxyde d'éthylène par le sulfure d'hydrogène avec une sélectivité en bêta-mercaptoéthanol supérieure à environ 99 % en poids sur la base d'un poids total de l'oxyde d'éthylène qui se convertit en bêta-mercaptoéthanol divisé par un poids total de l'oxyde d'éthylène qui se convertit en produit de réaction.

10. Procédé selon l'une quelconque des revendications 1 à 9, dans lequel le catalyseur solide comprend un copolymère de styrène-divinylbenzène avec des groupes actifs faiblement basiques comprenant des alkylamines.

11. Procédé selon l'une quelconque des revendications 1 à 10, dans lequel l'étape de mise en réaction comporte une conversion d'oxyde d'éthylène en produit de réaction qui est supérieure à environ 99 % en poids par rapport à un poids de l'oxyde d'éthylène qui convertit en produit de réaction divisé par un poids de l'oxyde d'éthylène alimenté au réacteur.

12. Procédé selon l'une quelconque des revendications 1 à 11, exécuté à une température dans une plage d'environ 30 °C à environ 80 °C.

13. Procédé selon l'une quelconque des revendications 1 à 12, dans lequel le lit fixe du réacteur a une température de lit moyenne en poids d'environ 65 °C à environ 70 °C.

14. Procédé selon l'une quelconque des revendications 1 à 13, dans lequel une vitesse spatiale horaire en poids de l'oxyde d'éthylène est dans la plage de 0,1 à 1h¹.

15. Procédé selon l'une quelconque des revendications 1 à 14, exécuté à une pression comprise dans une plage de 2068 kPa (300 psig) à 3447 kPa (500 psig).

16. Procédé selon l'une quelconque des revendications 1 à 15, exécuté à une pression de 3103 kPa (450 psig).

17. Procédé selon l'une quelconque des revendications 1 à 16, comprenant en outre :
la récupération d'une phase vapeur du sulfure d'hydrogène du réacteur ;
la condensation de la phase vapeur du sulfure d'hydrogène en une phase liquide ; et
le recyclage de la phase liquide du sulfure d'hydrogène au réacteur.
